(19) Europäisches Patentamt
European Patent Office
Office européen des brevets

(11) Veröffentlichungsnummer: **0 201 855**
**A2**

(12) # EUROPÄISCHE PATENTANMELDUNG

(21) Anmeldenummer: **86106213.1**

(22) Anmeldetag: **06.05.86**

(51) Int. Cl.⁴: **C07K 5/06**, C07K 1/14, C12P 21/02

(30) Priorität: **14.05.85 DE 3517361**

(43) Veröffentlichungstag der Anmeldung: **20.11.86 Patentblatt 86/47**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Platen, Martin, Dr.**
**Krautgartenweg 6**
**D-6000 Frankfurt am Main 50(DE)**
Erfinder: **Keller, Reinhold, Dr.**
**Wiesenweg 5**
**D-6232 Bad Soden am Taunus(DE)**
Erfinder: **Schlingmann, Merten, Dr.**
**Schneidhainer Strasse 32a**
**D-6240 Königstein/Taunus(DE)**

(54) **Verfahren zur Herstellung von Additionsverbindungen aus Dipeptiden und Aminen.**

(57) Das Dipeptid aus einer N-geschützten L-Asparaginsäure und L-Phenylalaninmethylester bildet mit Aminen schwer lösliche Additionsverbindungen, die sich leicht aus dem Reaktionsmedium abtrennen lassen.

EP 0 201 855 A2

## Verfahren zur Herstellung von Additionsverbindungen aus Dipeptiden und Aminen

Es ist bekannt, daß Proteasen nicht nur die hydrolytische Spaltung von Peptidbindungen, sondern auch die Knüpfung von Peptidbindungen aus Aminosäurederivaten unter Bildung von Dipeptiden katalysieren können. Die Deutsche Offenlegungsschrift 2 801 238 (US-Patentschriften 4 165 311, 4 256 836 und 4 436 925) beschreibt ein Verfahren zur Herstellung von Additionsverbindungen, bestehend aus einem N-geschützten Monoaminocarbonsäureester einerseits und einem Dipeptid andererseits, welches seinerseits aus diesem Monoaminocarbonsäureester und einer Monoaminodicarbonsäure in einer enzymatischen Veresterungsreaktion hergestellt wurde. Die Additionsverbindung ist im Reaktionsmedium unlöslich und wird abgetrennt. Das eigentliche Ziel dieser Umsetzung ist jedoch nicht die Herstellung der Additionsverbindung, sondern das ungeschützte Dipeptid selbst. Es sind in der Literatur, unter anderem auch in der obengenannten Deutschen Offenlegungsschrift, mehrere Verfahren beschrieben, die die Gewinnung des Dipeptids durch Zersetzung der Additionsverbindung zum Gegenstand haben.

Überraschend wurde nun gefunden, daß das Dipeptid, das in einer enzymatischen Reaktion der N-geschützten Asparaginsäure mit Phenylalaninmethylester entstanden ist, mit im Reaktionsmedium anwesenden Aminen schwer lösliche Salze bildet. Diese Reaktion läuft schneller ab als die Bildung von Additionsprodukten mit L-, D-oder D,L-Phenylalaninmethylester, wie sie in obengenannter Deutscher Offenlegungsschrift beschrieben wird.

Die Erfindung betrifft somit:

1. Eine neue Additionsverbindung eines Dipeptids mit einem Amin der allgemeinen Formel I,

$$R - NH - \underset{CH_3-CO_2^-}{CH} - \overset{O}{C} - NH - \underset{CH_2-C_6H_5}{CH} - CO_2CH_3 \cdot HY^+ \qquad I$$

in der R eine Aminoschutzgruppe und Y ein Amin ist, das frei von $\alpha$-Carboxygruppen oder Derivaten dieser Gruppe ist.

2. Verfahren zur Herstellung der Verbindung der obengenannten allgemeinen Formel I, in der R eine Aminoschutzgruppe und Y eine Aminoverbindung ist, durch Umsetzung einer Verbindung der allgemeinen Formel II

$$R - NH - \underset{CH_2 - COOH}{CH} - COOH \qquad II$$

mit L-Phenylalaninmethylester im wäßrigen Medium in Gegenwart einer Protease in einem pH-Bereich, in dem die Protease enzymatisch aktiv ist, dadurch gekennzeichnet, daß als Y ein Amin verwendet wird, das frei von $\alpha$-Carboxygruppen oder Derivaten dieser Gruppe ist.

In der weiteren Beschreibung wird die Erfindung genauer dargestellt bzw. in den Patentansprüchen definiert.

Die chemische Reaktion ist in der folgenden Gleichung dargestellt:

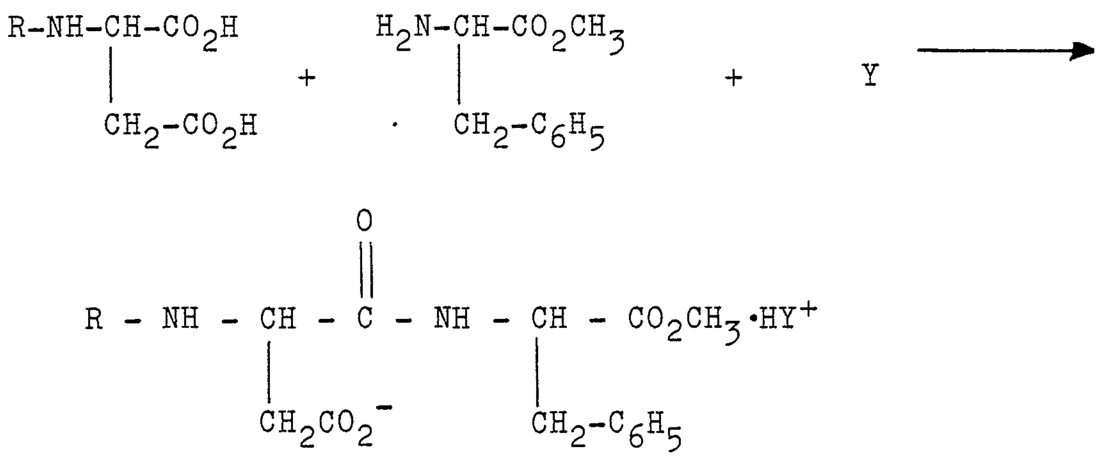

In dieser Reaktion ist es sinnvoll, die Aminogruppe der Asparaginsäure durch eine Aminoschutzgruppe zu schützen. Bevorzugt ist eine Gruppe vom Urethantyp, insbesondere eine Benzyloxycarbonylgruppe oder eine tertiär-Butyloxycarbonylgruppe.

Als organische Basen, in deren Gegenwart das Asparaginsäurederivat enzymatisch mit L-, D- und/oder D,L-Phenylalaninmethylester umgesetzt wird, können Amine der allgemeine Formel $H_{3-n}NR'_n$ eingesetzt werden, in der

a) n 1, 2 oder 3 ist und

b) R' für gleiche oder unterschiedliche Alkyl-, Cycloalkyl-, Aralkyl-oder Arylreste mit vorzugsweise bis zu 18 C-Atomen steht, von denen mindestens einer mindestens 6 C-Atome hat, wobei zwei oder drei der Reste R', gegebenenfalls unter Einschluß des N-Atoms, einen Ring bilden können, und

c) wobei die Carbonsäure-oder Mineralsäuresalze wasserlöslich sind.

Bevorzugt werden Verbindungen eingesetzt, in denen R' für gleiche oder verschiedene Alkyl-, Cycloalkyl-oder Aralkylreste mit 6 bis 8 C-Atomen steht, oder Verbindungen, in denen zwei oder drei Reste R' gemeinsam mit dem Stickstoffatom einen gesättigten oder ungesättigten Ring mit bis zu 10 C-Atomen bilden. Beispielhaft sollen hier Benzylamin, Dibenzylamin, Bis-(aminomethyl)-benzole wie 3-Aminomethyl-benzylamin, p-Aminophenyl-2-ethylamin, Chinolin und Dicyclohexylamin gennant werden. Ferner können anstelle von Aminen auch quartäre Ammoniumsalze der allgemeinen Formel $NR_4^+$ X verwendet werden, wobei X das Anion einer Säure ist.

Die Reaktion findet in einem wäßrigen Medium statt, das auch gepuffert sein kann. Der pH-Bereich der Pufferlösung entspricht dem Aktivitätsbereich der Enzyme. Bevorzugt sind Pufferkonzentrationen von 0,05 bis 1 M, insbesondere 0,1 bis 0,5 M. Das Asparaginsäurederivat und den Phenylalaninmethylester setzt man in Konzentrationen von 1 bis 25% ein, bezogen auf das Gewicht des Reaktionsmediums. Das Molverhältnis der Komponenten kann zwischen 20:1 und 1:50 liegen, vorzugsweise jedoch zwischen 5:1 und 1:5. Ein Überschuß an Phenylalaninester, beispielsweise in Form des D-Esters beim Einsatz des racemischen Ausgangsmaterials, stört überraschenderweise nicht, da -wie gefunden wurde -die Additionsreaktion mit dem Amin schneller verläuft als mit dem überschüssigen Phenylalaninester.

Das erfindungsgemäß eingesetzte Amin kann, bezogen auf das Dipeptid, in äquimolaren Mengen zugegeben werden. Bessere Ergebnisse erzielt man im allgemeinen bei Zugabe eines Überschusses, insbesondere eines 1-bis 50-fachen, vorzugsweise eines 2-bis 5-fachen molaren Überschusses. Das Amin kann als freie Verbindung oder als Salz, insbesondere als Hydrochlorid oder als Hydrogensulfat, verwendet werden.

Enzyme, die diese Reaktion katalysieren, sind Proteasen oder Metalloproteasen, wie z.B. $\alpha$-Chymotrypsin, Subtilisin, Thermolysin, Pepsin oder Trypsin, die einzeln oder auch in Mischungen eingesetzt werden können. Die Enzymmenge, die hierbei verwendet wird, liegt im Bereich von 5 U bis 30 000 U pro mmol der Ausgangsverbindungen, vorzugsweise von 50 U bis 10 000 U. Die Reaktion läuft bei einer Temperatur von 4 bis 50°C, vorzugsweise jedoch bei 35 bis 40°C und dem für das verwendete Enzym optimalen pH-Wert, im allgemeinen pH 5-7, ab. Die Inkubationszeit beträgt 2 bis 80 Stunden, wobei 20 bis 30 Stunden bevorzugt werden.

Die erfindungsgemäße Additionsverbindung wird dann nach an sich bekannten Methoden zur Gewinnung des freien Dipeptids zersetzt. Somit kann höchst vorteilhaft der L-Aspartyl-L-phenylalaninmethylester hergestellt werden.

Das erfindungsgemäße Verfahren läßt die Bildung der Additionsverbindung, im Vergleich zu dem in der genannten Deutschen Offenlegungsschrift beschriebenen Verfahren, schneller ablaufen. Weiterhin läßt sich das eingesetzte Amin aus der Additionsverbindung leicht wiedergewinnen, indem es bei der Spaltung der letzteren Verbindung als Salz einer Mineralsäure abgetrennt und aus dieser Lösung mit starken Basen freigesetzt wird. Bei den bekannten Verfahren besteht demgegenüber die Gefahr, daß der freigesetzte Phenylalaninester bei der Aufarbeitung verseift wird.

Die folgenden Beispiele geben eine weitere detaillierte Beschreibung der Erfindung. Die Prozentangaben beziehen sich auf das Gewicht.

Beispiel 1

1,79 g (8,31 mmol) L-Phenylalaninmethylesterhydrochlorid und 2,22 g (8, 31 mmol) L-N-Benzyloxycarbonylasparaginsäure werden unter Zugabe von 0,5 ml 7%iger wäßriger Ammoniaklösung in 20 ml bidestilliertem Wasser und 4 ml eines 1 M Natriumphosphatpuffers (pH 6) gelöst. Diese Mischung versetzt man anschließend mit 17 ml einer 14,7%igen wäßrigen Lösung von Dibenzylaminhydrogensulfat (8,45 mmol) und stellt mit einer 7%igen ammoniakalischen Lösung auf pH 6 ein. Nach Zugabe von 1325 U Thermolysin wird 24 Stunden bei 40°C inkubiert und anschließend die ausgefallene Additionsverbindung abgetrennt. 1 U Thermolysin hydrolysiert 1 mmol Z-Glycyl-Phenylalaninamid pro Minute bei 40°C und pH 7,5. Nach dem Umkristallisieren aus einem Gemisch aus Essigester, Methanol und Hexan im Verhältnis 8:1:1 - (v:v:v) erhält man das Additionsprodukt in einer Ausbeute von 62,5%.

Zur weiteren Charakterisierung werden folgende Analysenergebnisse aufgeführt:

Schmelzpunkt: 135-137°C

Elementaranalyse:

$C_{36}H_{39}N_3O_7$ (625,72) aus C 69,3, H 6,1, N 6,8

$^1$H-NMR ($CDCl_3$, 100 MHz):

$\delta$ = 7,59-7,11 (mehrere überlagerte m, 24H);

5,03 (s, 2H, $C_6H_5$ -$C\underline{H}_2$-O-CO-NH-),

4,60-4,30 (2 überlagerte m, 2H, -$CH_2$-$C\underline{H}$ =),

3,75 (s, 4H, ($C_6H_5$-$C\underline{H}_2$-NH),

3,61 (s, 3H, -$CO_2$-$C\underline{H}_3$),

3,49-3,31 (2 überlagerte m, 4H, -$C\underline{H}_2$-CH =)ppm

optischer Drehwert:

$[\alpha]_D^{25°}$ = 4,91 (c = 1, Methanol)

Beispiel 2

Man verfährt gemäß Beispiel 1, arbeitet jedoch bei einem pH-Wert von 7. Nach Aufarbeitung erhält man die Additionsverbindung in 71%iger Ausbeute.

Beispiel 3

Man verfährt gemäß Beispiel 1, stellt jedoch den pH auf einen Wert von 5 ein und inkubiert nach Enzymzugabe 70 Stunden bei 40°C. Nach Aufarbeitung erhält man die Additionsverbindung in 57%iger Ausbeute.

Beispiel 4

Man verfährt gemäß Beispiel 1, versetzt jedoch mit 70 ml einer 17,8%igen wäßrigen Lösung von Dibenzylaminhydrogensulfat (42,25 mmol) und inkubiert 70 Stunden bei 4°C. Nach Aufarbeitung erhält man die Additionsverbindung in 59%iger Ausbeute.

Beispiel 5

Man verfährt gemäß Beispiel 1, suspendiert jedoch die Aminosäurederivate in 16 ml bidestilliertem Wasser und 8 ml 1 M Phosphatpuffer und inkubiert nach Amin-und Enzymzugabe 9 Stunden bei 50°C. Nach Aufarbeitung erhält man die Additionsverbindung in 81%iger Ausbeute.

Beispiel 6

Man verfährt gemäß Beispiel 1, abweichend wird jedoch die genannte Menge Puffer durch die gleiche Menge bidestilliertes Wasser ersetzt und 265 U Thermolysin zugegeben. Nach Aufarbeitung erhält man die Additionsverbindung in 58%iger Ausbeute.

Beispiel 7

Man verfährt gemäß Beispiel 1, gibt jedoch 5 300 U Thermolysin zu dem Reaktionsgemisch und inkubiert 4 Stunden bei 40°C. Nach Aufarbeitung erhält man die Additionsverbindung in 83%iger Ausbeute.

Beispiel 8

Man verfährt zunächst gemäß Beispiel 1, gibt jedoch anstelle von Dibenzylaminhydrogensulfat 17 ml einer 11,5%igen wäßrigen Lösung von 3-Aminomethyl-benzylaminmonohydrogensulfat - (0,083 mmol) zu dem Reaktionsgemisch. Die weitere Reaktionsführung geschieht entsprechend Beispiel 7. Nach dem Umkristallisieren aus einem Gemisch aus Essigester, Methanol und Pentan im Verhältnis 8:4:2 (v:v:v) erhält man das Additionsprodukt in einer Ausbeute von 21,7%.

Zur weiteren Charakterisierung werden folgende Analysenergebnisse aufgeführt:

Schmelzpunkt: 116-119°C

Elementaranalyse:

$C_{30}H_{36}N_4O_7$(564,639) aus C 63,8, H 6,4, N 9,9

$^1$H-NMR ($d_6$-DMSO):

$\delta$ = 7,60-7,09 (mehrere m, 21 H, -$CO_2\underline{H}$, 2N$\underline{H}$, 2N$\underline{H}_2$),

5,00 (s, 2H, C$\underline{H}_2$-OCO-NH-),

4,5-4,23 (2 m, 2H, -$CH_2$-C$\underline{H}$=),

4,00 (s, 4H, $H_2$N-C $\underline{H}_2$-$C_6H_5$)

3,61 (s, 3H, -$CO_2C\underline{H}_3$)

3,45-3,17 (2 überlagerte m, 4H, -C$\underline{H}_2$-CH=)ppm

optischer Drehwert:

$[\alpha]_D^{25°}$ = 5,82 (c=1, Methanol)

Beispiel 9

Man verfährt gemäß Beispiel 1, gibt jedoch anstelle von Dibenzylaminhydrogensulfat 17 ml einer 8,6%igen wäßrigen Lösung von 2-(4-Aminophenyl)-ethylaminhydrochlorid (8,3 mmol) zu. Nach dem Umkristallisieren aus Essigester erhält man das Additionsprodukt mit einem Schmelzpunkt von 122-125°C in einer Ausbeute von 84%.

Beispiel 10

Man verfährt gemäß Beispiel 1, anstelle von 1,79 g L-Phenylalaninmethylesterhydrochlorid werden jedoch 0,179 g D,L-Phenylalaninmethylesterhydrochlorid (0,83 mmol) und die doppelte Aminmenge (16,9 mmol) in Form einer 29,4%igen wäßrigen Lösung des Dibenzylaminhydrogensulfats zugesetzt. Nach Aufarbeitung erhält man die Additionsverbindung in 69%iger Ausbeute.

Beispiel 11

Man verfährt gemäß Beispiel 1, anstelle von Dibenzylammoniumhydrogensulfat wird jedoch eine 11,2%ige wäßrige Lösung von Chinolinhydrogensulfat (8,42 mmol) eingesetzt. Nach dem Umkristallisieren erhält man das Additionsprodukt mit einem Schmelzpunkt von 127 bis 134°C in 67%iger Ausbeute.

Beispiel 12

Man verfährt in Anlehnung an Beispiel 1. 1,63 g (8,35 mmol) N-tertiär-Butyloxycarbonyl-L-asparaginsäure und 1,80 g (8,35 mmol) L-Phenylalaninmethylester werden gelöst und mit 17 ml einer 17,6%igen wäßrigen Lösung von Dibenzylaminhydrogensulfat (10,1 mmol) versetzt. Das Reaktionsgemisch wird bei einem pH von 7 über einen Zeitraum von 40 Stunden bei 35°C inkubiert. Nach dem Umkristallisieren aus einem Gemisch von Essigester, Methanol und Hexan im Verhältnis 10:2:5 (v:v:v) erhält man das Additionsprodukt mit einem Schmelzpunkt von 107 bis 112°C in einer Ausbeute von 56%.

## Ansprüche

1. Dipeptid-Amin-Additionsverbindung der allgemeinen Formel I

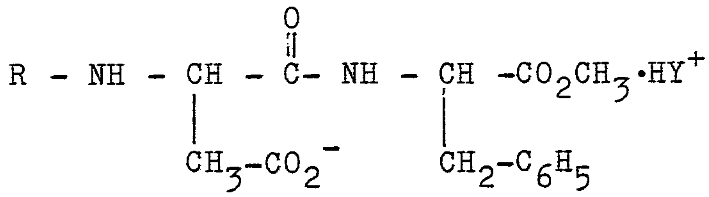

I

in der R eine Aminoschutzgruppe und Y ein Amin ist, das frei von α-Carboxygruppen oder Derivaten dieser Gruppe ist.

2. Additionsverbindung nach Anspruch 1, dadurch gekennzeichnet, daß R eine Schutzgruppe vom Urethantyp ist.

3. Additionsverbindung nach Anspruch 2, dadurch gekennzeichnet, daß R eine Benzyloxycarbonylgruppe oder eine tertiär-Butyloxycarbonylgruppe bedeutet.

4. Additionsverbindung nach einem oder mehreren der Ansprüche 1 bis 3, dadurch gekennzeichnet, daß Y ein Amin der Formel $H_{3-n}NR'_n$ bedeutet, in der

a) n 1, 2 oder 3 ist und

b) R' für gleiche oder unterschiedliche Alkyl-, Cycloalkyl-, Aralkyl-oder Arylreste steht, von denen mindestens einer mindestens 6 C-Atome hat, wobei zwei oder drei der Reste R', gegebenenfalls unter Einschluß des N-Atoms, einen Ring bilden können, und

c)wobei die Mineralsäuresalze oder die Carbonsäuresalze wasserlöslich sind.

5. Verbindung nach Anspruch 4, dadurch gekennzeichnet, daß R' für gleiche oder verschiedene Alkyl-, Cycloalkyl-oder Aralkylreste mit 6 bis 8 C-Atomen steht oder 2 oder 3 Reste R' gemeinsam mit dem Stickstoffatom einen gesättigten oder ungesättigten Ring mit bis zu 10 C-Atomen bilden.

6. Additionsverbindung nach Anspruch 4 oder 5, dadurch gekennzeichnet, daß das Amin ein Benzylamin, Dibenzylamin, Bis-(aminomethyl)-benzol, Aminophenyl-2-ethylamin, Chinolin oder Dicyclohexylamin ist.

7. Verfahren zur Herstellung der Verbindung der allgemeinen Formel I,

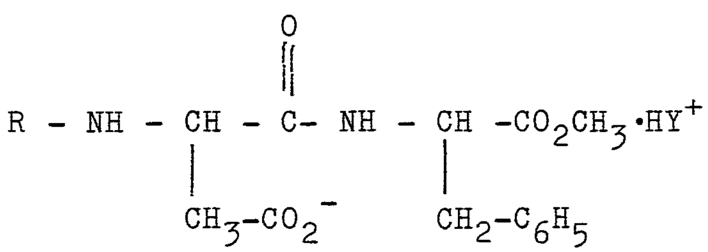

I

in der R eine Aminoschutzgruppe und Y ein Amin ist, durch Umsetzung einer Verbindung der allgemeinen Formel II,

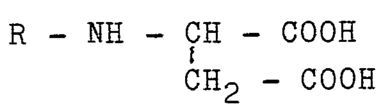

II,

in der R eine Aminoschutzgruppe ist, mit L-Phenylalaninmethylester im wäßrigen Medium in Gegenwart einer Protease in einem pH-Bereich, in dem die Protease enzymatisch aktiv ist, dadurch gekennzeichnet, daß als Y ein Amin verwendet wird, das frei von α-Carboxygruppen oder Derivaten dieser Gruppe ist.

8. Verfahren nach Anspruch 7, dadurch gekennzeichnet, daß als R eine Schutzgruppe vom Urethantyp verwendet wird.

9. Verfahren nach Anspruch 7 oder 8, dadurch gekennzeichnet, daß als R eine Benzyloxycarbonylgruppe oder eine tertiär-Butyloxycarbonylgruppe verwendet wird.

10. Verfahren nach einem oder mehreren der Ansprüche 7 bis 9, dadurch gekennzeichnet, daß als Y ein Amin der Formel $H_{3-n}NR'_n$ verwendet wird, wobei

a) n 1, 2 oder 3 ist und

b) R' für gleiche oder unterschiedliche Alkyl-, Cycloalkyl-, Aralkyl-oder Arylreste steht, von denen mindestens einer mindestens 6 C-Atome hat, wobei zwei oder drei der Reste R', gegebenenfalls unter Einschluß des N-Atoms, einen Ring bilden können, und

c) wobei die Mineralsäuresalze oder die Carbonsäuresalze wasserlöslich sind.

11. Verfahren nach einem oder mehreren der Ansprüche 7 bis 10, dadurch gekennzeichnet, daß R' für gleiche oder verschiedene Alkyl-, Cycloalkyl-oder Aralkylreste mit 6 bis 8 C-Atomen steht oder 2 oder 3 Reste R' gemeinsam mit dem Stickstoffatom einen gesättigten oder ungesättigten Ring mit bis zu 10 C-Atomen bilden.

12. Verfahren nach einem oder mehreren der Ansprüche 7 bis 11, dadurch gekennzeichnet, daß das Amin in einem Überschuß zugegeben wird.